# EUROPEAN PATENT APPLICATION

(11) **EP 2 093 212 A1**
(43) Date of publication of application: **26.08.2009**
(21) Application number: 08003028.1
(22) Date of filing: 19.02.2008
(51) Int. Cl.: C07C 59/72, A61K 31/202, A61K 31/335, C07C 59/54, C07C 229/56, C07D 313/00, C12P 7/42, C12P 13/00, C12P 17/08

(54) **Elansolids, novel natural metabolites of flexibacter and antibiotically active derivatives thereof**

(71) Applicant: Helmholtz-Zentrum für Infektionsforschung GmbH, 38124 Braunschweig (DE)
(72) Inventor: Gerth, Klaus Dr., D-38124 Braunschweig (DE); Steinmetz, Heinrich, D-38124 Braunschweig (DE); Höfle, Gerhard Prof. Dr., D-38124 Braunschweig (DE)
(74) Representative: Forstmeyer, Dietmar

(57) **Abstract**

The present invention describes Elansolids, a new class of novel anti-bacterial compounds of formula (I):

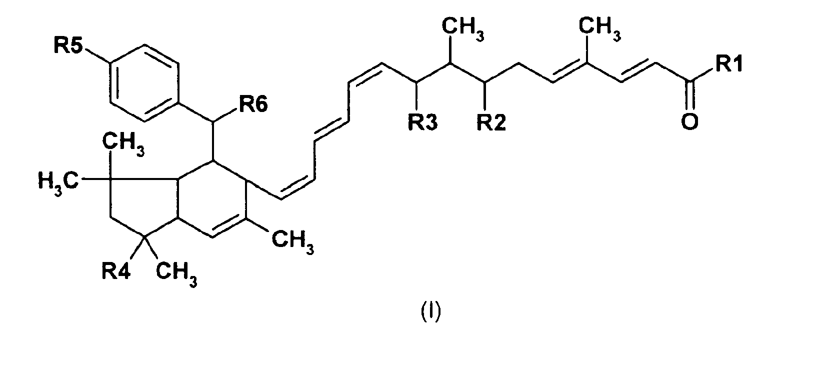

## Description

Over the past 20 years, there has been a rapid decline in the number of antibiotics approved annually. At the same time the fast emergence of resistance to most antibacterial drugs diminishes their effectiveness considerably and necessitates a constant supply of new antibiotics for effective treatment of infections. The discovery of novel antimicrobial agents has become an increasing challenge. Still, microbial natural products remain the most promising source of novel antibiotics. They own an element of structural complexity which is required for the inhibition of many bacterial protein targets.

The invention relates to novel natural secondary metabolites, the production of the metabolites by fermentation as well as a biological and a chemical derivatization providing a family of antibiotically active compounds.

The present invention describes new kinds of compounds having anti-bacterial activity.

The present invention relates to compounds (Elansolids) of the general formula (I): 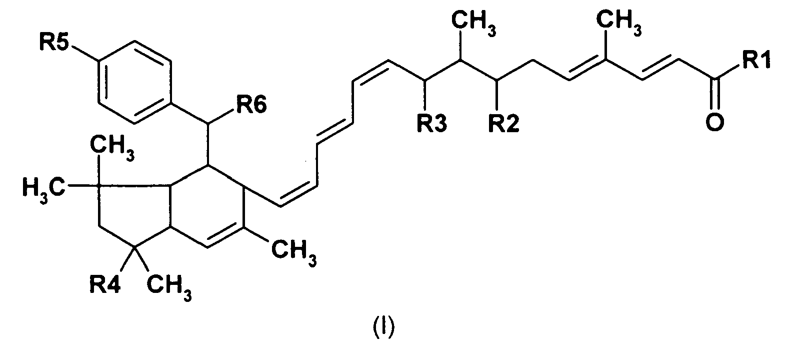 wherein
R1 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R2 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R3 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R4 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R5 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, and
R6 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, or
wherein R1 and R6 together are an oxygen atom, a sulfur atom or a NH group,
or a pharmacologically acceptable salt, solvate, hydrate or a pharmacologically acceptable formulation thereof.

The expression alkyl refers to a saturated, straight-chain or branched hydrocarbon group that contains from 1 to 20 carbon atoms, preferably from 1 to 12 carbon atoms, especially from 1 to 6 carbon atoms, for example a methyl, ethyl, propyl, iso-propyl, n-butyl, iso-butyl, tert-butyl, n-pentyl, n-hexyl, 2,2-dimethylbutyl or n-octyl group.

The expressions alkenyl and alkynyl refer to at least partially unsaturated, straight-chain or branched hydrocarbon groups that contain from 2 to 20 carbon atoms, preferably from 2 to 12 carbon atoms, especially from 2 to 6 carbon atoms, for example an ethenyl, allyl, acetylenyl, propargyl, isoprenyl or hex-2-enyl group. Preferably, alkenyl groups have one or two (especially one) double bond(s) and alkynyl groups have one or two (especially one) triple bond(s).

Furthermore, the terms alkyl, alkenyl and alkynyl refer to groups in which one or more hydrogen atoms have been replaced by a halogen atom (preferably F or Cl) such as, for example, a 2,2,2-trichloroethyl or a trifluoromethyl group.

The expression heteroalkyl refers to an alkyl, alkenyl (e.g. heteroalkenyl) or alkynyl group in which one or more (preferably 1, 2 or 3) carbon atoms have been replaced by an oxygen, nitrogen, phosphorus, boron, selenium, silicon or sulphur atom (preferably oxygen, sulphur or nitrogen). Examples for heteroalkyl groups are alkyloxy, alkyloxyalkyl, alkenyloxyalkyl, alkenyloxy, alkyloxyalkenyl, alkylamino, alkylaminoalkyl, dialkylamino, dialkylaminoalkyl, alkylthio or alkylthioalkyl groups. The expression heteroalkyl furthermore refers to a carboxylic acid or to a group derived from a carboxylic acid such as, for example, acyl, acyloxy, acyloxyalkyl, acylalkyl, alkoxycarbonyl, alkyloxycarbonylalkyl, carboxyalkylamide, alkylcarbonylamino, alkylcarbonylaminoalkyl, alkylaminocarbonyl, alkylaminocarbonylalkyl, or alkoxycarbonyloxy.

Examples of heteroalkyl groups are groups of formulae R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{A}-N(R^{b})-CO-O-Y^{a}-, R^{a-}N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-(preferred are R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}- and R^{a}-N(R^{b})-CO-Y^{a}-,), R^{a} being a hydrogen atom, a C₁-C₆alkyl, a C₂-C₆alkenyl or a C₂-C₆alkynyl group; R^{b} being a hydrogen atom, a C₁-C₆alkyl, a C₂-C₆alkenyl or a C₂-C₆alkynyl group; R^{c} being a hydrogen atom, a C₁-C₆alkyl, a C₂-C₆alkenyl or a C₂-C₆alkynyl group; R^{d} being a hydrogen atom, a C₁-C₆alkyl, a C₂-C₆alkenyl or a C₂-C₆alkynyl group and Y^{a} being a bond, a C₁-C₆alkylene, a C₂-C₆alkenylene or a C₂-C₆alkynylene group (preferrably, R^{a}, R^{b}, R^{c} and R^{d} are independently H or C₁-C₆ alkyl and Y^{a} is a bond or C₂-C₆alkylene), each heteroalkyl group containing at least one carbon atom and it being possible for one or more hydrogen atoms to have been replaced by fluorine or chlorine atoms. Specific examples of heteroalkyl groups are methoxy, trifluoromethoxy, ethoxy, n-propyloxy, isopropyloxy, *tert*-butyloxy, methoxymethyl, ethoxymethyl, methoxyethyl, methylamino, ethylamino, dimethylamino, diethylamino, isopropylethylamino, methylaminomethyl, ethylaminomethyl, diisopropylaminoethyl, enol ether, dimethylaminomethyl, dimethylaminoethyl, acetyl, propionyl, butyryloxy, acetyloxy, methoxycarbonyl, ethoxycarbonyl, N-ethyl-N-methylcarbamoyl and N-methylcarbamoyl. Further examples of heteroalkyl groups are nitrile, isonitrile, cyanate, thiocyanate, isocyanate, isothiocyanate and alkylnitrile groups.

The expression cycloalkyl refers to a saturated or partially unsaturated (for example cycloalkenyl), cyclic group that contains one or more rings (preferably 1 or 2), containing from 3 to 50 carbon atoms, preferably 3 to 14, further preferably from 3 to 10 (especially 3, 4, 5, 6 or 7) carbon atoms. The expression cycloalkyl refers furthermore to groups in which one or more hydrogen atoms have been replaced by fluorine, chlorine, bromine or iodine atoms or by OH, =O, SH, =S, NH₂, =NH or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetralin, cyclopentylcyclohexyl, fluorocyclohexyl or cyclohex-2-enyl group.

The expression heterocycloalkyl refers to a cycloalkyl group as defined above in which one or more (preferably 1, 2 or 3) ring carbon atoms have been replaced by an oxygen, nitrogen, silicon, selenium, phosphorus or sulphur atom (preferably oxygen, sulphur or nitrogen). A heterocycloalkyl group has preferably 1 or 2 ring(s) containing from 3 to 10 (especially 3, 4, 5, 6 or 7) ring atoms. The expression heterocycloalkyl refers furthermore to groups in which one or more hydrogen atoms have been replaced by fluorine, chlorine, bromine or iodine atoms or by OH, =O, SH, =S, NH₂, =NH or NO₂ groups. Examples are a piperidyl, piperazinyl, morpholinyl, urotropinyl, pyrrolidinyl, tetrahydrothiophenyl, tetrahydropyranyl, tetrahydrofuryl or 2-pyrazolinyl group and also lactams, lactones, cyclic imides and cyclic anhydrides.

The expression alkylcycloalkyl refers to groups containing both cycloalkyl and also alkyl, alkenyl or alkynyl groups in accordance with the above definitions, for example alkylcycloalkyl, cycloalkylalkyl, alkylcycloalkenyl, alkenylcycloalkyl and alkynylcyclo-alkyl groups. An alkylcycloalkyl group preferably contains a cycloalkyl group that contains one or two ring systems which build a scaffold containing from 3 to 10 (especially 3, 4, 5, 6 or 7) carbon atoms, and one or two alkyl, alkenyl or alkynyl groups having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, selenium, phosphorus or sulphur atom (preferably oxygen, sulphur or nitrogen). A heteroalkylcycloalkyl group preferably contains 1 or 2 ring systems having from 3 to 10 (especially 3, 4, 5, 6 or 7) ring atoms, and one or two alkyl, alkenyl, alkynyl or heteroalkyl groups having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl or Ar refers to an aromatic group that has one or more rings containing from 6 to 50 carbon atoms, preferably 6 to 14, further preferably from 6 to 10 (especially 6) carbon atoms. The expression aryl (or Ar) refers furthermore to groups in which one or more hydrogen atoms have been replaced by fluorine, chlorine, bromine or iodine atoms or by OH, SH, NH₂ or NO₂ groups. Examples are a phenyl, naphthyl, biphenyl, 2-fluorophenyl, anilinyl, 3-nitrophenyl or 4-hydroxyphenyl group.

The expression heteroaryl refers to an aromatic group that has one or more rings containing from 5 to 50 ring atoms, preferably 5 to 14, further preferably from 5 to 10 (especially 5 or 6) ring atoms, and contains one or more (preferably 1, 2, 3 or 4) oxygen, nitrogen, phosphorus or sulphur ring atoms (preferably O, S or N). The expression heteroaryl refers furthermore to groups in which one or more hydrogen atoms have been replaced by fluorine, chlorine, bromine or iodine atoms or by OH, SH, NH₂ or NO₂ groups. Examples are 4-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1 H-indene, tetralin, dihydronaphthalene, indanone, phenylcyclopentyl, cumene, cyclohexylphenyl, fluorene and indan. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl refers to an aralkyl group as defined above in which one or more (preferably 1, 2, 3 or 4) carbon atoms have been replaced by an oxygen, nitrogen, silicon, selenium, phosphorus, boron or sulphur atom (preferably oxygen, sulphur or nitrogen), that is to say to groups containing both aryl or heteroaryl and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (1 or 2 rings) containing from 5 or 6 to 10 carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced by oxygen, sulphur or nitrogen atoms.

Examples are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroarylalkylcycloalkyl, heteroarylalkylheterocycloalkenyl, heteroarylheteroalkylcycloalkyl, heteroarylheteroalkylcycloalkenyl and heteroarylheteroalkylheterocycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

As already stated above, the expressions cycloalkyl, heterocycloalkyl, alkylcycloalkyl, heteroalkylcycloalkyl, aryl, heteroaryl, aralkyl and heteroaralkyl refer to groups in which one or more hydrogen atoms of such groups have been replaced by fluorine, chlorine, bromine or iodine atoms or by OH, =O, SH, =S, NH₂, =NH or NO₂ groups.

The expression "optionally substituted" refers to groups in which one or more hydrogen atoms have been replaced by fluorine, chlorine, bromine or iodine atoms or by OH, =O, SH, =S, NH₂, =NH or NO₂ groups. This expression refers furthermore to groups that are substituted by unsubstituted C₁-C₆alkyl, C₂-C₆alkenyl, C₂-C₆alkynyl, C₁-C₆heteroalkyl, C₃-C₁₀cycloalkyl, C₂-C₉heterocycloalkyl, C₆-C₁₀aryl, C₁-C₉heteroaryl, C₇-C₁₂aralkyl or C₂-C₁₁heteroaralkyl groups.

Owing to their substitution, compounds of formula (I) may contain one or more centres of chirality. The present invention therefore includes both all pure enantiomers and all pure diastereoisomers and also mixtures thereof in any mixing ratio. The present invention moreover also includes all cis/trans-isomers of the compounds of the general formula (I) and also mixtures thereof. The present invention moreover includes all tautomeric forms of the compounds of formula (I).

Preferred are compounds of formula (I) wherein R1, R2, R3, R4, R5 and R6 are independently of each other a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group.

Further preferred are compounds of formula (I) wherein R1 is a hydroxyl group or a group of formula NH₂, OR7 or NHR8, wherein R7 and R8 are independently an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group.

Especially preferred are compounds of formula (I) wherein R1 is a hydroxyl group.

Further preferred are compounds of formula (I) wherein R2 is a hydroxyl group.

Further preferred are compounds of formula (I) wherein R3 is a hydroxyl group.

Further preferred are compounds of formula (I) wherein R4 is a hydroxyl group.

Further preferred are compounds of formula (I) wherein R5 is a hydroxyl group.

Further preferred are compounds of formula (I) wherein R6 is a group of formula NHR9 wherein R9 is an aryl, heteroaryl, aralkyl or heteroaralkyl (preferrably, aryl or heteroaryl, especially a phenyl or a pyridyl) group, all of which groups may optionally be substituted.

Further preferred are compounds of formula (I) wherein R6 is a group of formula OR10 wherein R10 is an aryl, heteroaryl, aralkyl or heteroaralkyl (preferrably, aryl or heteroaryl, especially a phenyl or a pyridyl) group, all of which groups may optionally be substituted.

Further preferred are compounds of formula (I) wherein R6 is a group of formula SR11 wherein R11 is an aryl, heteroaryl, aralkyl or heteroaralkyl (preferrably, aryl or heteroaryl, especially a phenyl or a pyridyl) group, all of which groups may optionally be substituted.

Moreover preferred are groups of formula (I) wherein R2 is a group of formula OC(=O)R2' or NHC(=O)R2" wherein R2' is an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group and R2" is an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group.

Moreover preferred are groups of formula (I) wherein R3 is a group of formula OC(=O)R3' or NHC(=O)R3" wherein R3' is an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group and R3" is an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group.

Moreover preferred are groups of formula (I) wherein R4 is a group of formula OC(=O)R4' or NHC(=O)R4" wherein R4' is an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group and R4" is an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group.

Moreover preferred are groups of formula (I) wherein R5 is a group of formula OC(=O)R5' or NHC(=O)R5" wherein R5' is an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group and R5" is an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group.

Moreover preferred are groups of formula (I) wherein R6 is a group of formula OC(=O)R6' or NHC(=O)R6" wherein R6' is an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group and R6" is an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group.

Further preferred are compounds of formula (I) wherein R1 and R6 together are an oxygen atom.

It is to be noted that the present invention also encompasses all possible combinations of all preferred embodiments.

Especially preferred, the compound of formula (I) has the following structure (Elansolid A):

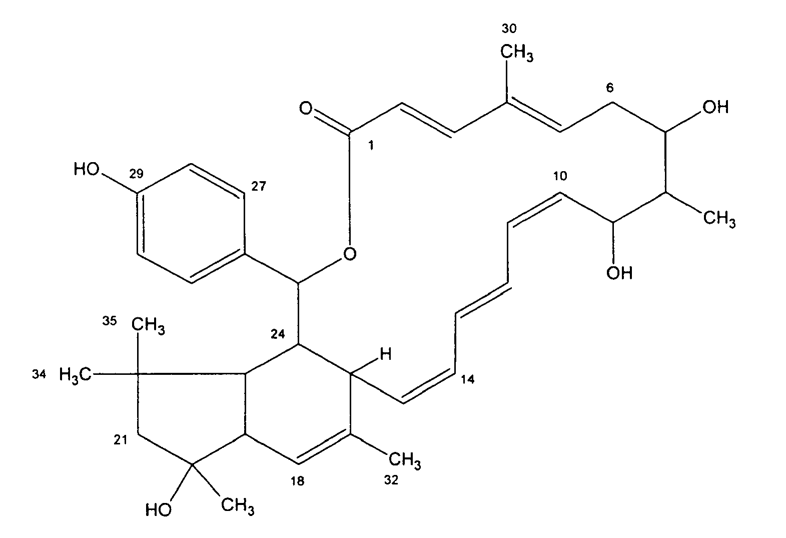

Further, especially preferred, the compound of formula (I) has the following structure (Elansolid B₁):

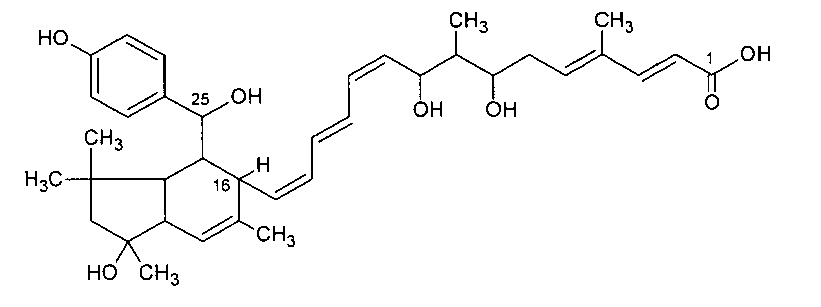

Further, especially preferred, the compound of formula (I) has the following structure (Elansolid B₂):

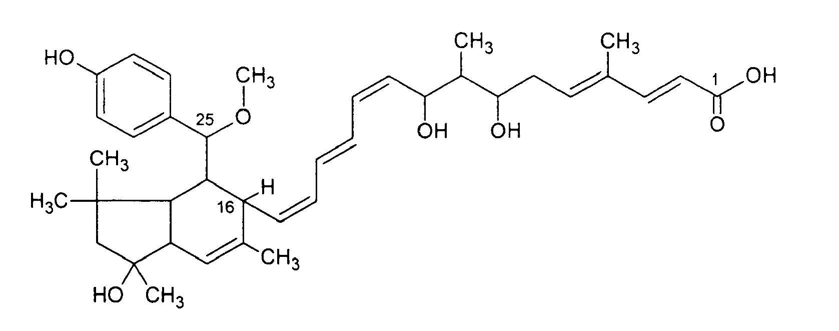

Further, especially preferred, the compound of formula (I) has the following structure (Elansolid C₁):

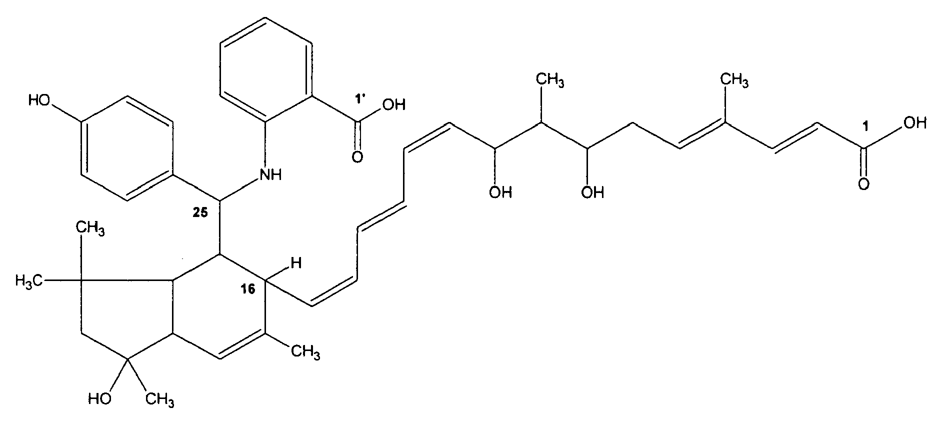

The therapeutic use of compounds of formula (I), their pharmacologically acceptable salts or solvates and hydrates and also formulations and pharmaceutical compositions also lie within the scope of the present invention, especially, the use of compounds of formula (I), their pharmacologically acceptable salts or solvates and hydrates and also formulations and pharmaceutical compositions for the treatment of bacterial infections as well as their use for the preparation of medicaments for the treatment of bacterial infections.

The pharmaceutically compositions according to the present invention comprise at least one compound of formula (I) as active ingredient and, optionally, carrier substances and/or adjuvants. Further, these pharmaceutically compositions may comprise other antimicrobial ingredients.

Examples of pharmacologically acceptable salts of the compounds of formula (I) are salts of physiologically acceptable mineral acids, such as hydrochloric acid, sulphuric acid and phosphoric acid, or salts of organic acids, such as methanesulphonic acid, p-toluenesulphonic acid, lactic acid, acetic acid, trifluoroacetic acid, citric acid, succinic acid, fumaric acid, maleic acid and salicylic acid. Further examples of pharmacologically acceptable salts of the compounds of formula (I) are alkali metal and alkaline earth metal salts such as, for example, sodium, potassium, lithium, calcium or magnesium salts, ammonium salts or salts of organic bases such as, for example, methylamine, dimethylamine, triethylamine, piperidine, ethylenediamine, lysine, choline hydroxide, meglumine, morpholine or arginine salts. Compounds of formula (I) may be solvated, especially hydrated. The hydration may take place, for example, during the preparation process or as a consequence of the hygroscopic nature of the initially anhydrous compounds of formula (I). When the compounds of formula (I) comprise asymmetric C-atoms, they may be present either in the form of achiral compounds, diastereoisomeric mixtures, mixtures of enantiomers or in the form of optically pure compounds.

The pro-drugs to which the present invention also relates consist of a compound of formula (I) and at least one pharmacologically acceptable protecting group which will be removed under physiological conditions, such as, for example, an alkoxy-, aralkyloxy-, acyl- or acyloxy group, such as, for example, an ethoxy, benzyloxy, acetyl or acetyloxy group.

The present invention relates also to the use of those active ingredients in the preparation of medicaments. In general, compounds of formula (I) are administered either individually, or in combination with any other desired therapeutic agent, using the known and acceptable methods. Such therapeutically useful agents may be administered, for example, by one of the following routes: orally, for example in the form of dragées, coated tablets, pills, semi-solid substances, soft or hard capsules, solutions, emulsions or suspensions; parenterally, for example in the form of an injectable solution; rectally in the form of suppositories; by inhalation, for example in the form of a powder formulation or a spray; transdermally or intranasally. For the preparation of such tablets, pills, semi-solid substances, coated tablets, dragées and hard gelatine capsules, the therapeutically usable product may be mixed with pharmacologically inert, inorganic or organic pharmaceutical carrier substances, for example with lactose, sucrose, glucose, gelatine, malt, silica gel, starch or derivatives thereof, talcum, stearic acid or salts thereof, skimmed milk powder, and the like. For the preparation of soft capsules, pharmaceutical carrier substances such as, for example, vegetable oils, petroleum, animal or synthetic oils, wax, fat and polyols may be used. For the preparation of liquid solutions and syrups, pharmaceutical carrier substances such as, for example, water, alcohols, aqueous saline solution, aqueous dextrose, polyols, glycerol, vegetable oils, petroleum and animal or synthetic oils may be used. For suppositories, pharmaceutical carrier substances such as, for example, vegetable oils, petroleum, animal or synthetic oils, wax, fat and polyols may be used. For aerosol formulations, compressed gases that are suitable for this purpose, such as, for example, oxygen, nitrogen and carbon dioxide may be used. The pharmaceutically acceptable agents may also comprise additives for preserving and stabilising, emulsifiers, sweeteners, flavourings, salts for altering the osmotic pressure, buffers, encapsulation additives and antioxidants.

Combinations with other therapeutic agents may comprise other antimicrobial ingredients.

For the prevention and/or treatment of bacterial infections, the dose of the biologically active compound according to the invention may vary within wide limits and may be adjusted to individual requirements. Generally, a dose of from 10 mg to 4000 mg per day is suitable, a preferred dose being from 50 to 3000 mg per day. In suitable cases, the dose may also be below or above the stated values. The daily dose may be administered as a single dose or in a plurality of doses. A typical individual dose contains approximately 50 mg, 100 mg, 250 mg, 500 mg, 1 g or 2 g of the active ingredient.

Also the following methods for producing compounds (Elansolids) of formula (I) lie within the scope of the present invention.

Compounds of formula (I) (elansolids) can be produced by culturing a selected gliding bacterium of the genus *Chitinophagha* spec. [*Flexibacter spec.*] It is understood that the production of elansolid like substances is not limited to the use of the particular organism described herein, which is given for illustrative purpose only. The invention also includes the use of any mutants which are capable of producing elansolid type substances including natural mutants as well as artificial mutants which can be produced from the described organism by conventional means such as irradiation or treatment with mutagens. Also genetically manipulated mutants and the expression of the gene cluster responsible for biosynthesis in the producer strain or by heterologous expression in host strains are part of the invention.

Compounds of formula (I) can be attained by feeding nucleophilic precursors to growing cultures of FxGBF13 (mutasynthesis) or by chemical reactions (e.g. derivatization of Elansolid A or other Elansolids).

The microorganisms, which can be used for the production of elansolid A and the elansolid B derivatives, belong to genus *Chitinophaga* [*Flexibacter*]. The isolate Fx GBF13 used for production is closely related to *Chitinophage sancti* [*Flexibacter sancti*] according to 16S rDNA sequencing. The strain has been deposited at the German Collection of Microorganisms and Cell Cultures (DSMZ) Braunschweig, Germany under the accession number DSM 21134.

Elansolids are produced in liquid culture, by growing in media containing one or several different carbon sources, and one or different nitrogen sources. Also salts are essential for growth and production. Suitable carbon sources are different mono-, di-, and polysaccharides like glucose, sucrose, lactose or carbon from amino acids like peptones. Nitrogen sources are ammonium, nitrate, urea, chitin or nitrogen from amino acids. The following inorganic ions support the growth or are essential in synthetic media:Ca-ions, Mg-ions, Fe-ions, Mn-ions, Zn-ions, K-ions, sulfate-ions, Cl-ions, phosphate-ions.

Temperatures for growth and production are between 10°C to 40 °C, preferred temperatures are between 15°C and 37 °C, especially between 20°C to 25°C.

The test cultures were grown on CAS agar plates of the following composition: Casitone Difco, 10 g/l; MgSO₄ x 7 H₂O, 1 g/l; CaCl₂ x 2 H₂O 1 g/l; HEPES buffer 50 mM; agar 15 g/l; pH adjusted to 7.0. The cultures were incubated at 20°C.

### Production of Elansolids by fermentation in bioreactors

### Example: Fermentation in complex medium.

### Precultures in Erlenmeyer flasks:

Medium composition: Casitone Difco, 10 g/l; MgSO₄ x 7 H₂O, 1 g/l; CaCl₂ x 2 H₂O, 1 g/l; Hepes buffer 50 mM; pH adjusted to 7.0.
2 x 500 ml of the above medium in 1 I Erlenmeyer flasks are autoclaved and inoculated with 10% of a FxGBF13 preculture. Incubation for 4 days at 20 °C in a rotary incubator at 300 rpm.

### Fermentation in a 10 I bioreactor:

The medium used was identical with the medium in shake flasks. 10 I of fermentation broth were inoculated with 1 I of preculture. The fermentation was run at 20 °C with a stirrer speed of 100 r.p.m. and an aeration of 0,1 vvm for 4 days.

### Production in 100 I bioreactor:

The medium was identical with that in the 10 I bioreactor but without HEPES buffer. For continuous adsorption of the elansolids 2 % (v/v) of XAD 16 adsorber resin (Rohm and Haas, Frankfurt/M) was added before autoclaving.

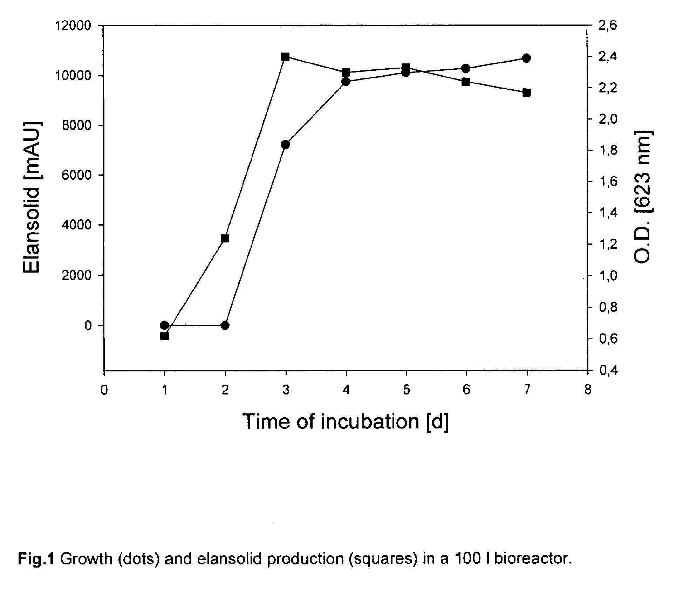

Fermentation parameters: The total fermentation volume was 70 I. The aeration rate was 0,1 vvm. The initial stirrer velocity was 100 r.p.m. and a pO₂ of 20% was maintained by regulation of the stirrer velocity. A pH of 7.0 was maintained by regulation with 10% sulphuric acid solution and 10% solution of KOH. Foam formation was inhibited by addition of silicone antifoam (Tegosipon, Goldschmidt AG, Essen). After 7 days of fermentation the XAD resin was harvested. (Fig. 1)

### Isolation of Elansolid A

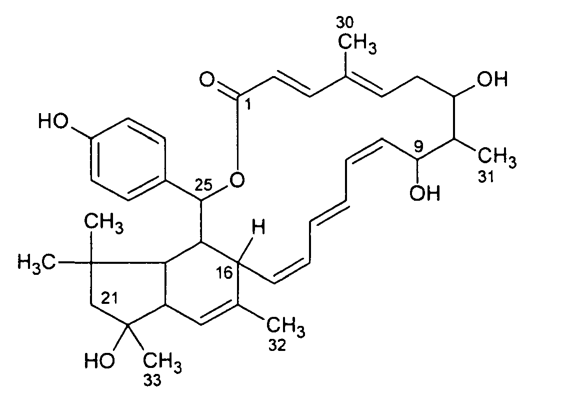

The XAD 16 resin of the above fermentation was harvested by sieving, washed with water, and eluted in a chromatography column with 9 L of acetone/methanol (1:1). The organic solvent was removed from the eluate by evaporation, and the remaining water was extracted three times with ethyl acetate. The combined ethyl acetate portions were dried with sodium sulphate and evaporated to dryness. The oily residue was partitioned between methanol and n-heptane. Evaporation of the methanol yielded 14.0 g raw extract, which was separated in portions of 800 mg by RP-MPLC [column 48×3 cm (Kronlab) ODS AQ 120 16 C18; eluent A: CH₃CN/H₂O 4:6, 50 mM NH₄OAc, pH 5.5; eluent B: CH₃CN/H₂O 50:50, 50 mM NH₄OAc, pH 5.5, gradient 100% A to 100% B in 360 min, flow: 12 ml/min; UV detection at 254 nm]. The Elansolid A containing fraction was evaporated to yield 32 mg/run. The product was further purified by RP-HPLC [column 250×21 mm, Nucleodur 100-7-C18 (Macherey+Nagel); eluent A: CH₃CN/H₂O 1:1, 50 mM NH₄OAc, pH 5.5, eluent B: CH₃CN/H₂O 7:3, 50 mM NH₄OAc, pH 5.5, gradient 100% A to 100% B in 30 min, flow 15 ml/min; UV detection at 254 nm] to yield 18 mg Elansolid A. The total yield from the 70 L fermenter was 315 mg.

*Elansolid A*: C₃₇H₄₈O₆ MW = 588.8 HRMS: [EI]⁺ calcd. 588.3451, found 588.3399 UV(MeOH): λₘₐₓ (Ig ε) = 225 (4,23), 257 sh (4,56), 270 (4,71), 280 (470) nm. IR (KBr): υ = 3419, 2961, 2929, 1693, 1616, 1515, 1249 cm⁻¹.

TLC (silica gel, UV detection at 254 nm, dichlormethane/methanol 85:15) R_{f} = 0.47. Analytical RP-HPLC: column 125×2 mm Nucleodur 120 5 µm C18 (Macherey Nagel), solvent A: water/acetonitrile 95/5, 5 mM NH₄OAc, pH 5.5, solvent B = water/acetonitrile 5/95, 5 mM NH₄OAc, pH 5.5; gradient from 10% B to 100% B in 30 min, 10 min 100% B, flow 0.3 ml/min. Rₜ = 20,4 min.

**Table 1. ¹H- and ¹³C NMR data of Elansolid A in DMSO-d₆ [¹H 600 MHz, ¹³C 150 MHz; internal standard solvent signal δ_{H}/δ_{C} = 2.50/39.51]**

| **At room temperature** | | | | | | | **At 60°C** | | |
|---|---|---|---|---|---|---|---|---|---|
| H | δ | m | *J* [Hz] | C | δ | m | δ | m | *J* [Hz] |
| - | | | | 1 | 165.7 | s | | | |
| 2 | 5.59 | d | 15.5 | 2 | 115.3 | d | 5.64 | d | 15.9 |
| 3 | 7.48 | d | 15.5 | 3 | 149.6 | d | 7.50 | d | 15.9 |
| - | - | - | - | 4 | 133.3 | d | - | - | - |
| 5 | 6.25 | t | 6.6 br | 5 | 141.1 | d | 6.26 | dd | 7.9, 5.8 br |
| 6b | 2.34 | d | 14 br | 6 | 32.9 | t | 2.41 | ddd | 15.5, 5.8, 2.5 |
| 6a | 2.25 | dt | 14, 8.0 | | | | 2.29 | dt | 15.9, 7.9 br |
| 7 | 3.62 | m | br | 7 | 71.0 | d | 3.67 | dddd | 8.0, 7.9, 5.3, 2.5 br |
| 8 | 1.61 | ddq | | 8 | 45.1 | d | 1.61 | ddq | 8.0, 4.0, 6.8 |
| 9 | 4.75 | m | br | 9 | 66.6 | d | 4.82 | ddd | 8.8, 4.8, 4.0 br |
| 10 | 5.53 | dd | 10.8, 9.8 | 10 | 134.4 | d | 5.55 | dd | 10.6, 8.8 |
| 11 | 5.99 | dd | 11.2, 10.6 | 11 | 128.2 | d | 5.99 | t | 10.6, 11.5 |
| 12 | 6.57 | dd | 14.4, 11.0 | 12 | 127.5 | d | 6.59 | dd | 14.3, 11.5 |
| 13 | 6.14 | dd | 14.3, 11.4 | 13 | 130.2 | d | 6.15 | dd | 14.3, 11.3 |
| 14 | 6.22 | dd | 11.4, 10.6 | 14 | 125.4 | d | 6.23 | t | 11.3, 10.6 |
| 15 | 5.74 | dd | 10.6, 10.6 | 15 | 134.4 | d | 5.74 | t | 10.6 |
| 16 | 2.85 | dd | 10.6, 2.5 | 16 | 40.2 | d | 2.89 | dd | 10.6, 3.8 br |
| - | - | - | - | 17 | 134.3 | s | - | - | - |
| 18 | 5.45 | | s br. | 18 | 122.8 | d | 5.47 | s | br. (dq 2, 1.1) |
| 19 | 2.58 | dd | 12.0,2.5 | 19 | 54.9 | d | 2.60 | d | 12.0 br |
| - | - | - | - | 20 | 74.3 | s | | - | - |
| 21b | 1.69 | d | 13.6 | 21 | 59.7 | t | 1.72 | d | 14.0 |
| 21a | 1,55 | d | 13.6 | | | | 1.58 | d | 14.0 |
| - | - | - | - | 22 | 37.2 | s | - | - | - |
| 23 | 1.75 | dd | 12, 11.2 | 23 | 44.1 | d | 1.77 | t | 12.0 |
| 24 | 2.17 | dd | 11.2, 4.0, 2.6 | 24 | 47.1 | d | 2.17 | ddd | 11.3, 3.8, 2.7 |
| 25 | 5.93 | d | 2.6 | 25 | 74.4 | d | 5.96 | d | 2.7 |
| - | - | - | - | 26 | 130.1 | s | - | - | - |
| 27 | 6.86 | d | 8.5 | 27 | 126.3 | d | 6.88 | d | 8.7 |
| 28 | 6.62 | d | 8.5 | 28 | 114.7 | d | 6.64 | d | 8.7 |
| - | - | - | - | 29 | 156.2 | s | - | - | - |
| 30 | 1.67 | s | | 30 | 11.9 | q | 1.72 | s | |
| 31 | 0.89 | d | 6.8 | 31 | 10.2 | q | 0.89 | d | 6.8 |
| 32 | 1.41 | s | br | 32 | 20.9 | q | 1.44 | dd | 2.3, 1.1 |
| 33 | 1.00 | s | | 33 | 26.4 | q | 1.03 | s | |
| 34 | 1.21 | s | | 34 | 23.8 | q | 1.23 | s | |
| 35 | 0.92 | s | | 35 | 29.9 | q | 0.89 | s | |
| 7 OH | 4.86 | d | 4.5 | | | | 4.78 | d | 5.3 |
| 9 OH | 4.70 | d | 3.8 | | | | 4.56 | d | 4.9 |
| 20 OH | 4.48 | s | | | | | 4.30 | s | |
| 29 OH | 9.33 | s | | | | | 9.33 | s | |

| | | | | | | | | | |
|---|---|---|---|---|---|---|---|---|---|
| Signals for 27 and 28 have double intensity; | | | | | | | | | |

### Derivatives of elansolid A at carbon C-25 (R6)

The enhanced reactivity of lactones toward nucleophiles makes hydrolysis their preferred reaction which is effected under variety of reaction conditions including reagents, catalysts, solvents and temperature. The chemoselectivity allows reactions of multifunctional compounds, very often of rather complicated structure. The ring cleavage occurs e.g. under basic (alkali metal hydroxides or alkoxides, tertiary amines) or acidic (Bronsted or Lewis acids) conditions. The effective cleavage of the lactone ring occurs also in reactions with nitrogen nucleophiles which include ammonia, primary and secondary amines and hydrazine derivatives, as commonly used reagents. Sulphur and selenium derivatives are prepared in reactions of lactones with S- and Se-nucleophiles.

The effective nucleophilic reducing agents include metal hydrides, of which lithium aluminium hydride is the most frequent used reagent. Metalloorganic reagents effectively cleave lactones, while new carbon-carbon bonds are formed. Other reducing agents like sodium borohydride and other complex boron hydrides can also be used.

Under opening of the lactone ring system derivatives of elansolid A at position C25 (R6) can be obtained by reaction with nucleophilic (preferably low molecular weight) chemicals.

Under neutral aqueous conditions the residue can be a hydroxy group (R6 = OH, elansolid Be) from reaction with water or a methoxy group formed in presence of methanol (R6 = OCH₃, elansolid B₂). Under alkaline conditions in presence of ammonium ions, the residue is an ammonium group (R6 = NH₂, elansolid B₃).

Of especial interest is the group of elansolid C derivatives, which result from a nucleophilic attack of more complex chemical structures. Such derivatives show especially high inhibitory activity against pathogens. Examples are derivatives which result from nucleophilic attack by substituted aromatic amines (like elansolid C₁). The new ring system at R6 can e.g. be aromatic or heteroaromatic and may also be optionally substituted. The ring system can be substituted at different positions and with different substituents such as e.g. COOH, COOMe, SO₃H, SO₂NH₂, CH=CHCOOH, halogen atoms, OH, bi- and polycylic aryl or heteroaryl groups.

Derivatives at C-25 (R6) can be obtained by mutasynthesis, i.e. by feeding the growing cultures of FxGBF13 with the respective chemicals like e.g. methyl-2-aminobenzoic acid, 2-aminopyridine-3-carboxylic acid, anthranilic acid, p-aminobenzoic acid, 4-aminobenzamide, sulfanilic acid, sulfanilamide or by chemical derivatization of purified elansolid A with e.g. mercapto benzoic acid, salicilic acid, 4-hydroxybenzoic acid, 4-hydroxybencoic acid methyl ester, hydroxycinnamic aicd.

### Isolation of Elansolid B₁

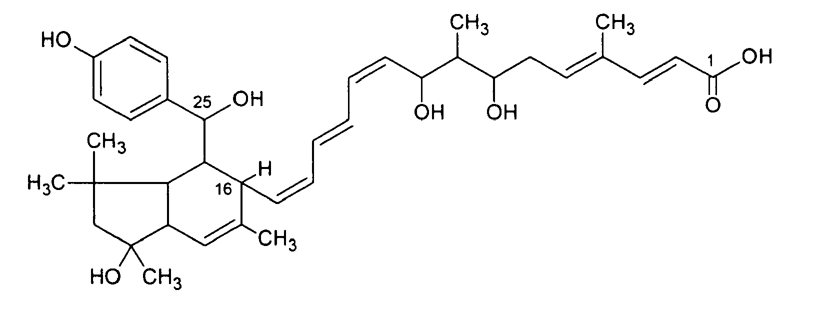

Wet cell mass and adsorber resin Amberlite XAD-16 (0.2 L) from a fermentation (10 L) of strain FXGBF13 was harvested by centrifugation and extracted with 3 portions of acetone (1.7 L each). The aqueous mixture which remained after evaporation of the acetone was extracted with ethyl acetate (1 L in all). The organic solution was dried with sodium sulphate and evaporated to yield about 6.8 g of raw product. 3 g of the residue was separated by silica gel flash chromatography (column 8.5×4 cm, silica gel ICN 60 63-200 µm), solvents CH₂Cl₂ (0.5 L), CH₂Cl₂/CH₃OH 95:5 (0.75 L), CH₂Cl₂/CH₃OH 85:15 (0.75 L). According to TLC analyses the fractions containing Elansolid B₁ were combined, and evaporated to dryness (yield 1.56 g). 110 mg of the enriched product was separated further by RP-HPLC [column 250×21 mm, Nucleodur 100 10 C18, Macherey Nagel, solvent 70% acetonitrile in water with 50 mM NH₄OAc, detection UV absorption at 235 nm] to yield 15 mg of Elansolid B₁.

### Elansolid B₁:

MF C₃₇H₅₀O₇, MW = 606.8, ESIMS: m/z 605.3 [M-H]⁻.
UV: λ [nm] (Ig ε) = 262 (sh 4.61), 272 (4.65), 282 (4.69), 296 (4.51)
TLC (silica gel 254 nm, dichloromethane/methanol 85:15): R_{f} = 0.28
HPLC: column 125×2 mm Nucleodur 120 5 µm C18 (Macherey Nagel), solvent A: water/acetonitrile 95/5 plus 5 mM NH₄OAc, pH 5.5, solvent B: water/acetonitrile 5/95 plus 5 mM NH₄OAc, pH 5.5) gradient: 10% B to 100% B in 30 min, 10 min 100% B, flow 0.3 mL/min. Rₜ = 10.1 min.

**Table 2. NMR data of Elansolid B₁ (R = OH) in DMSO-d₆, [¹H 600 MHz, ¹³C 150 MHz, internal ref. ¹H/¹³C δ = 2.50/39.51 ppm]**

| H | δ_{H} | m | *J* [Hz] | C | δ_{C} | m |
|---|---|---|---|---|---|---|
| - | | | | 1 | 167.9 | s |
| 1OH | 12.0 | s | v. br | | | |
| 2 | 5.71 | d | 15.8 | 2 | 116.1 | d |
| 3 | 7.21 | d | 15.8 | 3 | 149.0 | d |
| - | | | | 4 | 133.0 | s |
| 5 | 6.10 | dd | 7.1, 7.1 br | 5 | 139.9 | d |
| 6a | 2.24 | ddd | 15.5, 8, 7 | 6 | 33.3 | t |
| 6b | 2.40 | ddd | 15.5, 7, 2.9 | | | |
| 7 | 3.60 | ddd | 8, 7, 3.3 br | 7 | 71.3 | d |
| 8 | 1.56 | dt | 11.4, 3.3 br | 8 | 44.9 | d |
| 9 | 4.70 | dd | 8.4, 3.7 | 9 | 66.9 | d |
| 10 | 5.43 | dd | 11.2, 8.4 | 10 | 133.7 | d |
| 11 | 5.90 | dd | 11.5, 11.2 | 11 | 128.5 | d |
| 12 | 6.50 | dd | 13.9, 12.1 | 12 | 126.7 | d |
| 13 | 6.00 | dd | 13.9, 10.5 | 13 | 130.8 | d |
| 14 | 5.95 | dd | 10.5, 10.5 | 14 | 124.4 | d |
| 15 | 5.58 | dd | 10.6, 10.5 | 15 | 135.3 | d |
| 16 | 2.70 | dd | 10.5, 3.9 | 16 | 39.5 | d |
| - | | | | 17 | 134.6 | s |
| 18 | 5.40 | s. | br | 18 | 122.6 | d |
| 19 | 2.49 | d | ∼12 | 19 | 54.5 | d |
| - | | | | 20 | 74.5 | d |
| 21a | 1.69 | d | 13.6 | 21 | 60.2 | t |
| 21b | 1.58 | d | 13.6 | | | |
| - | | | | 22 | 37.5 | s |
| 23 | 1.79 | dd | 12.4, 11.4 | 23 | 43.9 | d |
| 24 | 1.91 | dt | 11.4, 3.9 | 24 | 48.0 | d |
| 25 | 5.00 | s. | br | 25 | 71.3 | d |
| - | | | | 26 | 135.2 | s |
| 25OH | 4.43 | s. | br | | | |
| 27* | 7.01 | d | 8.4 | 27 | 127.0 | d |
| 28* | 6.59 | d | 8.4 | 28 | 114.3 | d |
| 29OH | 9.11 | s | | 29 | 155.5 | s |
| 30 | 1.76 | s | | 30 | 12.3 | q |
| 30 | 0.87 | d | 7.0 | 31 | 10.2 | q |
| 32 | 1.34 | s | br. | 32 | 21.1 | q |
| 33 | 0.99 | s | | 33 | 26.2 | q |
| 34/35 | 1.19 | s | | 34 | 24.3 | q |
| 34/35 | 1.19 | s | | 35 | 31.4 | q |

| | | | | | | |
|---|---|---|---|---|---|---|
| Signals for 27 and 28 have double intensity; | | | | | | |

### Isolation of Elansolid B₂

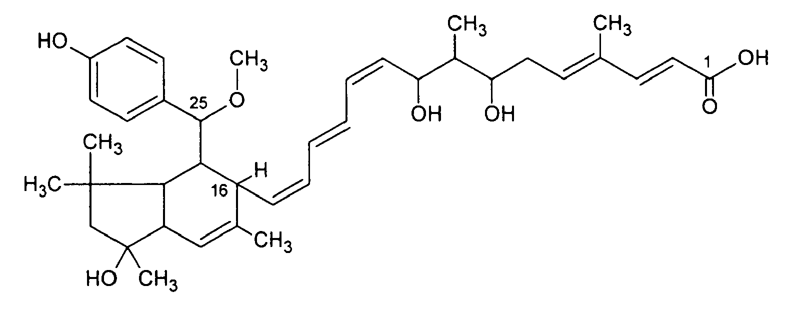

Amberlite XAD16 resin (1.2 L) was harvested by sieving from 70 L of fermentation broth of *Flexibacter* FXGBF13 and was washed with water. After eluting the resin with 9 L of methanol, the organic solvent was evaporated. The remaining water was extracted three times with ethyl acetate. The organic layer was concentrated to yield 15 g of raw extract.

The residue was resolved in methanol and partitioned with n-hexane. The methanol layer was concentrated to yield 14.1 g of crude extract.

650 mg of the extract was further separated by MPLC [column 48x3 cm, Kronlab ODS AQ 120, 16 µm, C18, eluent: acetonitrile/50 mM NH₄OAc buffer 48:52, flow 16 mL/min, UV detection at 254 nm). The Elansolid B₂ containing fraction was evaporated to yield 74.9 mg.

### Elansolid B₂:

MF C₃₈H₅₂O₇, MW = 620.8
ESIMS: *m*/*z* 619.2 [M-H]⁻.
UV: λ [nm] (Ig ε) = 229 (4.30), 259 (sh 4.60), 270 (4.66), 281 (4.68), 294 (4.53).
IR (KBr): ν = 3390, 3023, 2964, 2926, 2890, 1688, 1615, 1512, 1201 cm⁻¹.
TLC (silica gel; UV 254 nm, dichlormethane/methanol 85:15): R_{f} = 0.40.
HPLC (column 125×2 mm Nucleodur 120 5 µm C18 (Macherey Nagel), solvent A: water/acetonitrile 95/5, 5 mM NH₄OAc, pH 5.5, solvent B: water/acetonitrile 5/95, 5 mM NH₄OAc, pH 5.5, gradient from 100% A to 100% B in 30 min, 10 min 100% B, flow 0.3 mL/min): Rₜ = 15.8 min.

**Table 3. NMR data of elansolid B₂ in DMSO-d₆ [¹H 600 MHz, ¹³C 150 MHz, internal ref. ¹H/¹³C δ = 2.50/39.51 ppm]**

| H | δ | m | J [Hz] | C | δ |
|---|---|---|---|---|---|
| - | - | | | 1 | 167.8 |
| 1OH | 12.0 | s | v. br | | |
| 2 | 5.68 | d | 15.7 | 2 | 116.0 |
| 3 | 7.18 | d | 15.7 | 3 | 148.9 |
| - | - | | | 4 | 132.9 |
| 5 | 6.09 | dd | 7.0 | 5 | 139.8 |
| 6ₐ | 2.22 | ddd | 15.3, 7.9, 7.9 | 6 | 33.1 |
| 6_{b} | 2.40 | ddd | 15.3, 7.2, 2.6 | | |
| 7 | 3.59 | m | | 7 | 71.2 |
| 8 | 1.55 | m | | 8 | 44.9 |
| 9 | 4.68 | m | | 9 | 66.9 |
| 10 | 5.45 | dd | 10.6, 8.3 | 10 | 134.0 |
| 11 | 5.98 | dd | 11.9, 10.6 | 11 | 128.4 |
| 12 | 6.55 | dd | 14.5, 11.9 | 12 | 126.9 |
| 13 | 6.19 | dd | 14.5, 11.5 | 13 | 130.8 |
| 14 | 5.97 | dd | 11.5, 10.8 | 14 | 124.1 |
| 15 | 5.54 | dd | 10.8, 10.3 | 15 | 134.9 |
| 16 | 2.74 | dd | 10.3, 3.4 | 16 | 40.0 |
| - | - | | | 17 | 134.5 |
| 18 | 5.38 | s | br | 18 | 122.6 |
| 19 | 2.46 | d | 12.4 br | 19 | 54.6 |
| - | - | | | 20 | 74.3 |
| 21ₐ | 1.59 | d | 13.7 | 21 | 60.1 |
| 21_{b} | 1.69 | d | 13.7 | | |
| - | - | | | 22 | 37.4 |
| 23 | 1.76 | m | (with 30) | 24 | 44.2 |
| 24 | 1.82 | dt | 11.3, 3.4 br | 23 | 48.2 |
| 25 | 4.51 | d | 2.5 | 25 | 82.5 |
| - | - | | | 26 | 130.1 |
| 27 | 6.92 | d | 8.6 | 27 | 127.9 |
| 28 | 6.61 | d | 8.6 | 28 | 114.7 |
| - | - | | | 29 | 156.1 |
| 30 | 1.75 | s | | 30 | 12.2 |
| 31 | 0.88 | d | 7.2 | 31 | 10.1 |
| 32 | 1.35 | s | | 32 | 21.1 |
| 33 | 0.97 | s | | 33 | 26.2 |
| 34 | 1.18 | s | | 34 | 24.2 |
| 35 | 1.11 | s | | 35 | 30.7 |
| 36 OMe | 3.00 | s | | 36 | 55.9 |
| 7 OH | 4.73 | d | 5.6 | | |
| 9 OH | 4.69 | s | br | | |
| 20 OH | 4.44 | s | | | |
| 29 OH | 9.30 | s | | | |

| | | | | | |
|---|---|---|---|---|---|
| Signals for 27 and 28 have double intensity; signal C-16 under solvent signal; | | | | | |

### Elansolid C₁ (a C25 anthranilic acid derivative)

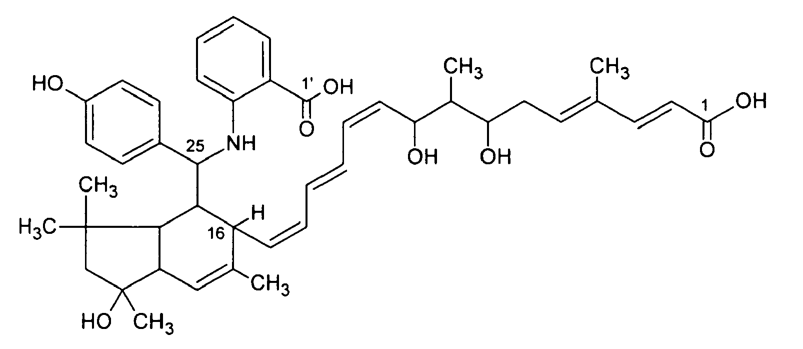

### Production by Mutasynthesis:

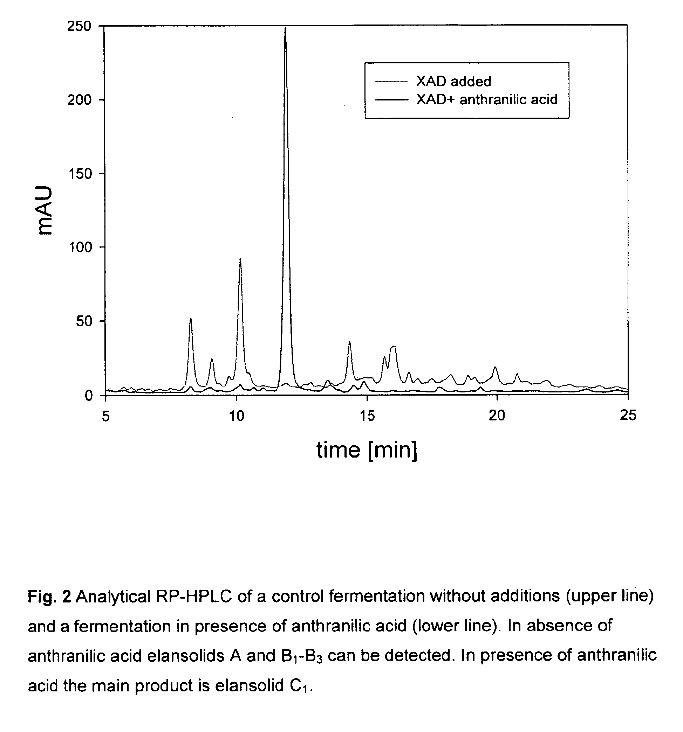

The fermentation is performed in a 15 I bioreactor with 10 I of complex culture medium as describe for elansolid A production. After sterilization anthranilic acid - dissolved in a small volume of methanol - is added to give a final concentration of 100 µg/l. The fermentation process is run as described above. After 5 days of fermentation at 20°C a XAD 16 suspension (150 ml) is added. After one hour of stirring the resin is harvested. Elansolid C₁ is observed as main component. (Fig. 2)

### Isolation of Elansolid C₁

From 10 L of fermentation broth adsorber resin XAD 16 (150 mL) was harvested by sieving (200 µm pore size). The resin was washed with water and eluted with 1 L of acetone in 3 steps. After evaporation of the acetone, the residual water layer was extracted three times with ethyl acetate. The organic layer was dried with sodium sulphate, filtrated, and concentrated in vacuo to give a crude extract of 489 mg.

The extract was separated by RP-HPLC in 10 runs [column 250×21 mm, Nucleodur 100-7-C18, (Macherey-Nagel), eluent acetonitrile/50 mM NH₄Ac buffer, gradient from 20% to 70% acetonitrile in 40 min, flow 15 mL/min, UV detection at 254 nm]. After evaporation of the Elansolid C containing fraction the water layer was extracted with ethyl acetate to yield Elansolid C₁ (71 mg).
Elansolid C₁: MF C₄₄H₅₅NO₈, MW = 725.9.
(+)-HR-ESIMS: found 748.3830, calcd. 748.3825 [M+Na]⁺.
UV: λ [nm] (Ig ε) = 220 (4.35), 259 (4.45), 273 (sh), 282 (sh), 346 (3.48). IR (KBr): ν = 3377, 2963, 1686, 1614, 1511, 1237 cm⁻¹.
TLC (silica gel, UV detection at 254 nm, CH₂Cl₂/MeOH 85:15): Rf = 0.48.
HPLC: column 2×125 mm Nucleodur 120 5 µm C18 (Macherey Nagel), solvent A: water/acetonitrile 95/5, 5 mM NH₄Ac, pH 5.5, solvent B: water/acetonitrile 5/95, 5 mM NH₄Ac, pH 5.5, gradient: 10% B to 100% B in10 min, 10 min 100% B, flow 0.3 mL/min. Rₜ = 6.6 min.

**Table 4. NMR data of Elansolid C₁ in d₆-DMSO [¹H 600 MHz, ¹³C 150 MHz; internal standard: solvent signal δ_{H/C} = 2.50/39.51 ppm]**

| RT | | | | 60°C | | | | 60°C | RT |
|---|---|---|---|---|---|---|---|---|---|
| H | δ | m | J [Hz] | δ | m | J [Hz] | C | δ | δ |
| - | - | | | - | | | 1 | 167.8 | 167.4 |
| 1OH | 12.3 | s | v. br | n.o. | | | | | |
| 2 | 5.68 | d | 15.8 | 5.65 | d | 15.6 | | 2 116.1 | 115.9 |
| 3 | 7.16 | d | 15.5 | 7.17 | d | 15.6 | 3 | 148.8 | 148.5 |
| - | - | | | - | | | 4 | 132.8 | 132.6 |
| 5 | 6.06 | dd | 7.0 7.4 | 6.06 | dd | 7.7,7.2 | 5 | 139.7 | 139.2 |
| 6b | 2.39 | ddd | 15.1, 7, 3.8 | 2.37 | ddd | 15.4, 7.2, 3.7 | 6 | 33.2 | 33.3 |
| 6a | 2.24 | ddd | 15.1, 7.8, 7.4 | 2.23 | ddd | 15.4, 8.0, 7.7 br | | | |
| 7 | 3.59 | ddd | 7.8, 6.9 3.8 | 3.68 | ddt | 8.0, 7.0, 3.7 | 7 | 71.2 | 71.4 |
| 8 | 1.58 | ddq | 6.9, 4.0, 6.8 | 1.59 | ddq | 7.0, 3.8, 7.0 | 8 | 45.0 | 44.6 |
| 9 | 4.70 | dd | 9.0, 4.0^{[a]} | 4.71 | dd | 8.6, 3.8 | 9 | 66.8 | 67.0 |
| 10 | 5.48 | dd | 10.5, 9.0 | 5.49 | dd | 10.6, 8.6 | 10 | 134.9 | 134.3 |
| 11 | 5.90 | dd | 10.5, 11.9 | 5.91 | dd | 11.7, 10.6 | 11 | 128.0 | 127.9 |
| 12 | 6.56 | dd | 14.3 11.9 | 6.59 | dd | 14.4, 11.7 | 12 | 128.4 | 128.2 |
| 13 | 5.97 | dd | 14.2 11.8 | 5.98 | dd | 14.4, 11.6 | 13 | 129.9 | 129.7 |
| 14 | 6.15 | dd | 11.8, 10.8 | 6.16 | dd | 11.6, 10.8 | 14 | 128.6 | 128.4 |
| 15 | 5.78 | dd | 10.8 10.6 | 5.78 | t | 10.8 | 15 | 131.9 | 131.7 |
| 16 | 2.77 | dd | 10.6 3.9 br | 2.78 | dd | 10.8, 3.8 | 16 | 40.6 | 40.5 |
| - | - | | | - | | | 17 | 134.5 | 134.2 |
| 18 | 5.47 | s | br | 5.47 | d | 1.8 br | 18 | 122.5 | 122.2 |
| 19 | 2.63 | d | 12.0 br | 2.64 | d | 11.7 br | 19 | 54.7 | 54.6 |
| - | - | | | - | | | 20 | 74.1 | 73.9 |
| 21b | 1.69 | d | 13.6 | 1.69 | d | 13.6 | 21 | 59.8 | 59.9 |
| 21a | 1.53 | d | 13.6 | 1.53 | d | 13.6 | 22 | 37.5 | 37.2 |
| 23 | 1.86 | dd | 12.0, 11.7 | 1.85 | t | 11.9 br | 23 | 44.3 | 44.2 |
| 24 | 2.35 | dt | 11.7, 3.8 | 2.36 | dt | 11.7, 4.2, 3.8 | 24 | 47.7 | 47.6 |
| 25 | 4.68 | dd | 3.8, 4.2^{[a]} | 4.58 | d | 4.2, 5.5 br | 25 | 56.9 | 56.8 |
| 25NH | 8.60 | d | 4.2 br | 8.52 | d | 5.5 br | | - | |
| - | - | | | - | | | 26 | 131.1 | 130.9 |
| 27 | 6.96 | d | 7.6 | 7.96 | d | 8.1 | 27 | 127.7 | 127.5 |
| 28 | 6.62 | d | 8.3 | 6.63 | d | 8.1 | 28 | 114.9 | 114.8 |
| - | - | | | - | | | 29 | 155.9 | 155.7 |
| 30 | 1.68 | s | | 1.69 | s | | 30 | 12.0 | 11.8 |
| 31 | 0.87 | d | 6.8 | 0.86 | d | 7.0 | 31 | 10.2 | 10.0 |
| 32 | 1.39 | s | br | 1.39 | s | br | 32 | 20.8 | 20.5 |
| 33 | 1.03 | s | | 1.03 | s | | 33 | 26.2 | 25.9 |
| 34 | 1.23 | s | | 1.23 | s | | 34 | 24.0 | 23.8 |
| 35 | 0.75 | s | | 0.75 | s | | 35 | 30.6 | 30.3 |
| - | - | | | - | | | 1' | 170.1 | 169.6 |
| - | - | | | - | | | 2' | 110.4 | 110.3 |
| 3' | 7.76 | dd | 7.8, 1 | 7.76 | dd | 7.8, 1.6 | 3' | 131.5 | 131.2 |
| 4' | 6.44 | dd | 7.8, 7.4 | 6.45 | dd | 7.8, 7.4 | 4' | 114.0 | 113.7 |
| 5' | 7.09 | dd | 8.3, 7.4, 1 | 7.10 | dddd | 8.3, 7.4, 1.6 | 5' | 134.2 | 133.8 |
| 6' | 6.11 | d | 8.3 | 6.12 | d | 8.3 | 6' | 111.8 | 111.6 |
| - | | | | | | | 7' | 149.9 | 149.7 |

| | | | | | | | | | |
|---|---|---|---|---|---|---|---|---|---|
| ^{[a]} the signals of H9 and H25 overlapp partially; Signals for 27 and 28 have double intensity; signal C-16 under solvent signal; | | | | | | | | | |

### Elansolids of formula (I) by chemical synthesis:

1) Reaction of Elansolid A with 2-aminobenzoic acid in ammonia/methanol 1:1, pH 8, at RT for 16 h yields Elansolid C₁.
2) Reaction of Elansolid A with 2-mercaptobenzoic acid under the same conditions yields the corresponding mercaptobenzoic acid derivative.

The reactions can be monitored by RP-HPLC-MS.

The derivatisation of Elansolid A (or other derivatives like e.g. Elansolid B₁, B₂ or C₁) at groups R2-R5 can be carried out using usual chemical reactions and synthesis methods known to a person skilled in the art.

### Characterization of the antibiotic activity of elansolid C1

### Antibiotic spectrum against gram positive bacteria

| Test organism | Elansolid C1 Inhibition- zone [mm] |
|---|---|
| *Arthrobacter simplex* | 25 |
| *Bacillus polymyxa* | 20 |
| *Bacillus subtilis* | - |
| *Bacillus thuringiensis* | 17 |
| *Brevibact. ammoniagenes* | 17 |
| *Corynebact. fascians* | 26 |
| *E. coli* CG | - |
| *E. coli ECA-res.* | - |
| *H 747* | - |
| *Klebsiella spec.* | - |
| *Micrococcus luteus* | 27 |
| *Mycobact. chitae* | 15 |
| *Mycobact. diernhoferi* | - |
| *Mycobact. smegmatis* | - |
| *Nocardia flava* | 14 |
| *Salmonella thyphymurium* | - |
| *Staph. aureus* | 11 |
| *Streptococcus faecalis* | 14 |

Elansolid C₁ exhibits a bactericidal activity against selected gram-positive bacteria. The minimal inhibition concentration (MIC) is 0.4 µg/ml against *Micrococcus luteus* and 1.7 µg/ml against a clinical isolate of a multiresistant *S. aureus* (MRS3).

## Claims

1. Compound of general formula (I): 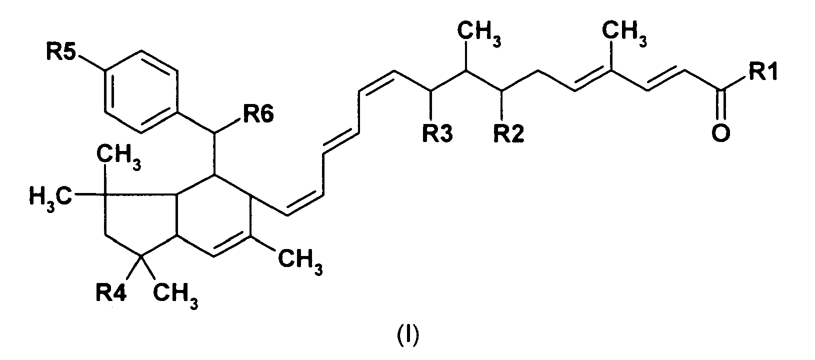 wherein
R1 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R2 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R3 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R4 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group,
R5 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, and
R6 is a hydrogen atom, a halogen atom, a hydroxy, an amino, a mercapto, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group, or
wherein R1 and R6 together are an oxygen atom, a sulfur atom or a NH group,
or a pharmacologically acceptable salt, solvate, hydrate or a pharmacologically acceptable formulation thereof.

2. Compund according to claim 1, wherein R1 is a hydroxyl group or a group of formula NH₂, OR7 or NHR8, wherein R7 and R8 are independently an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group.

3. Compund according to claim 1 or 2, wherein R2, R3, R4 and R5 are hydroxyl groups.

4. Compund according to anyone of the preceding claims wherein R6 is a group of formula NHR9 wherein R9 is an optionally substituted aryl, heteroaryl, aralkyl or heteroaralkyl group or a group of formula OR10 wherein R10 is an optionally substituted aryl, heteroaryl, aralkyl or heteroaralkyl group or a group of formula SR11 wherein R11 is an optionally substituted aryl, heteroaryl, aralkyl or heteroaralkyl group.

5. Compound according to claim 1 or 3, wherein R1 and R6 together are an oxygen atom.

6. A compound having one of the following formulas:

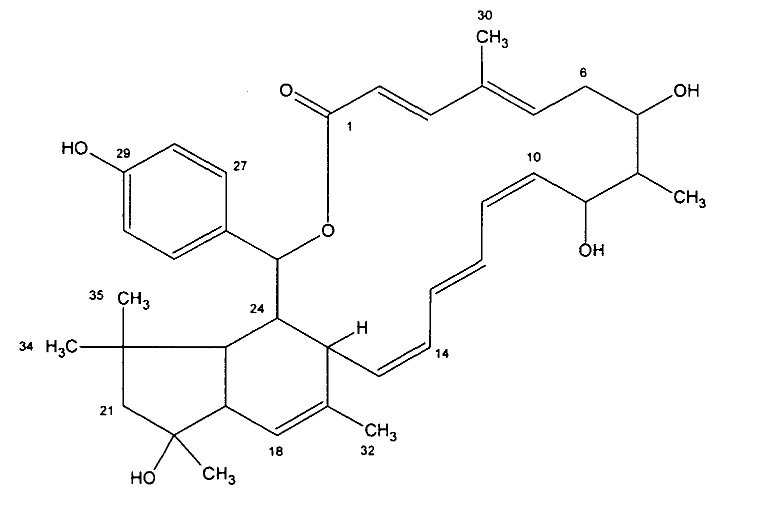

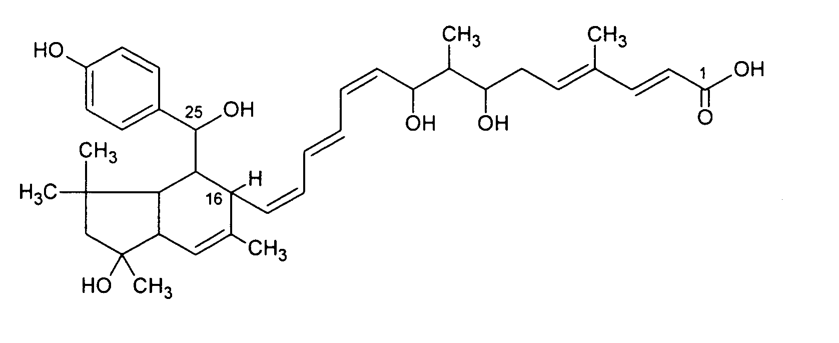

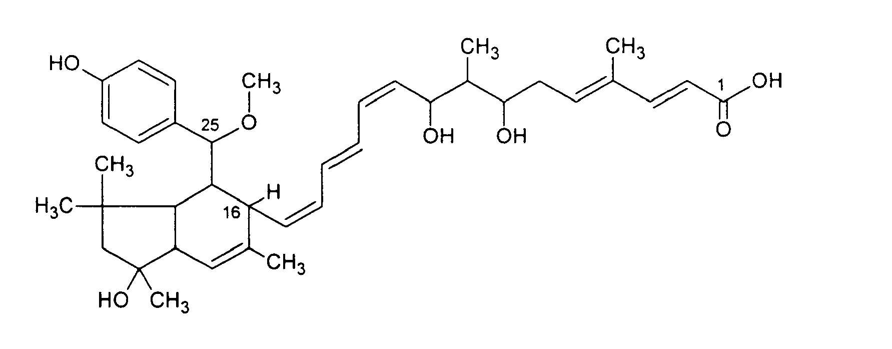

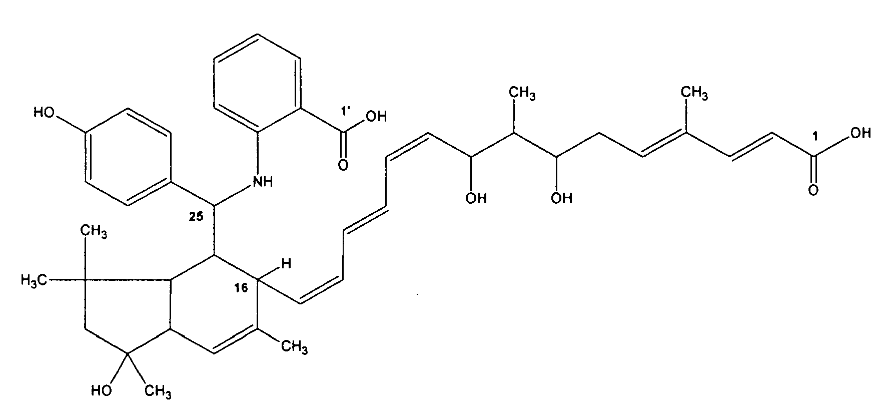

7. Compound (Elansolid A) having the following parameters: C₃₇H₄₈O₆ MW = 588.8 HRMS: [EI]⁺ calcd. 588.3451, found 588.3399; UV(MeOH): λₘₐₓ (Ig ε) = 225 (4,23), 257 sh (4,56), 270 (4,71), 280 (470) nm; IR (KBr): ν = 3419, 2961, 2929, 1693, 1616, 1515, 1249 cm⁻¹; TLC (silica gel, UV detection at 254 nm, dichlormethane/methanol 85:15) R_{f} = 0.47; Analytical RP-HPLC: column 125×2 mm Nucleodur 120 5 µm C18 (Macherey Nagel), solvent A: water/acetonitrile 95/5, 5 mM NH₄OAc, pH 5.5, solvent B = water/acetonitrile 5/95, 5 mM NH₄OAc pH 5.5; gradient from 10% B to 100% B in 30 min, 10 min 100% B, flow 0.3 ml/min. Rₜ = 20,4 min and ¹H- and ¹³C NMR data as shown in table 1.

8. Compound (Elansolid B₁) having the following parameters: C₃₇H₅₀O₇, MW = 606.8, ESIMS: *m*/*z* 605.3 [M-H]⁻; UV: λ [nm] (Ig ε) = 262 (sh 4.61), 272 (4.65), 282 (4.69), 296 (4.51); TLC (silica gel 254 nm, dichloromethane/methanol 85:15): R_{f} = 0.28; HPLC: column 125×2 mm Nucleodur 120 5 µm C18 (Macherey Nagel), solvent A: water/acetonitrile 95/5 plus 5 mM NH₄OAc, pH 5.5, solvent B: water/acetonitrile 5/95 plus 5 mM NH₄OAc, pH 5.5) gradient: 10% B to 100% B in 30 min, 10 min 100% B, flow 0.3 mL/min. Rₜ = 10.1 min and ¹H- and ¹³C NMR data as shown in table 2.

9. Compound (Elansolid B₂) having the following parameters: C₃₈H₅₂O₇, MW = 620.8; ESIMS: *m*/*z* 619.2 [M-H]⁻; UV: λ [nm] (Ig ε) = 229 (4.30), 259 (sh 4.60), 270 (4.66), 281 (4.68), 294 (4.53); IR (KBr): ν = 3390, 3023, 2964, 2926, 2890, 1688, 1615, 1512, 1201 cm⁻¹; TLC (silica gel; UV 254 nm, dichlormethane/methanol 85:15): R_{f} = 0.40; HPLC (column 125×2 mm Nucleodur 120 5 µm C18 (Macherey Nagel), solvent A: water/acetonitrile 95/5, 5 mM NH₄OAc, pH 5.5, solvent B: water/acetonitrile 5/95, 5 mM NH₄OAc, pH 5.5, gradient from 100% A to 100% B in 30 min, 10 min 100% B, flow 0.3 mL/min): Rₜ = 15.8 min and ¹H- and ¹³C NMR data as shown in table 3.

10. Compound (Elansolid C₁) having the following parameters: C₄₄H₅₅NO₈, MW = 725.9; (+)-HR-ESIMS: found 748.3830, calcd. 748.3825 [M+Na]⁺; UV: λ [nm] (Ig ε) = 220 (4.35), 259 (4.45), 273 (sh), 282 (sh), 346 (3.48); IR (KBr): ν = 3377, 2963, 1686, 1614, 1511, 1237 cm⁻¹; TLC (silica gel, UV detection at 254 nm, CH₂Cl₂/MeOH 85:15): Rf = 0.48; HPLC: column 2×125 mm Nucleodur 120 5 µm C18 (Macherey Nagel), solvent A: water/acetonitrile 95/5, 5 mM NH₄Ac, pH 5.5, solvent B: water/acetonitrile 5/95, 5 mM NH₄Ac, pH 5.5, gradient: 10% B to 100% B in 10 min, 10 min 100% B, flow 0.3 mL/min. Rₜ = 6.6 min and ¹Hand ¹³C NMR data as shown in table 4.

11. Pharmaceutical composition comprising a compound according to anyone of the preceding claims and optionally carrier substances and/or adjuvants.

12. Use of a compound or a pharmaceutical composition according to anyone of the preceding claims for the treatment of bacterial infections.

13. A method of producing a compound according to anyone of claims 1 to 10 **characterized in that** the strain DSM 21134 is used for the production.

14. Strain DSM 21134.
